# EUROPÄISCHE PATENTSCHRIFT

(11) **EP 1 311 264 B1**
(45) Veröffentlichungstag und Bekanntmachung des Hinweises auf die Patenterteilung: **27.06.2007**
(21) Anmeldenummer: 01969532.9
(22) Anmeldetag: 27.07.2001
(51) Int. Cl.: A61K 31/425, A61P 3/10, C07D 291/08

(54) **SUBSTITUIERTE UND UNSUBSTITUIERTE BENZOOXATHIAZOLE SOWIE DARAUS ABGELEITETE VERBINDUNGEN**
SUBSTITUTED AND NON-SUBSTITUTED BENZOOXATHIAZOLES AND COMPOUNDS DERIVED THEREFROM
BENZOOXATHIAZOLES SUBSTITUES ET NON SUBSTITUES ET COMPOSES DERIVES DE CEUX-CI

(30) Priorität: 09.08.2000 DE 10038709
(43) Veröffentlichungstag der Anmeldung: 21.05.2003
(73) Patentinhaber: Sanofi-Aventis Deutschland GmbH, 65929 Frankfurt am Main (DE)
(72) Erfinder: PETRY, Stefan, 65929 Frankfurt (DE); BARINGHAUS, Karl-Heinz, 61200 Wölfersheim (DE); HÖLDER, Swen, 60954 Frankfurt am Main (DE); MUELLER, Guenter, 65843 Sulzbach (DE)
(86) Internationale Anmeldenummer: PCT/EP2001/008704
(87) Internationale Veröffentlichungsnummer: WO 2002/011722

(56) Entgegenhaltungen:
- WO-A-00/05216
- WO-A-98/32438
- WO-A-98/32439
- WO-A-99/11264
- WO-A-99/36069
- DATABASE CROSSFIRE BEILSTEIN [Online] Beilstein Institut zur Förderung der Chemischen Wissenschaften, Frankfurt am Main, DE; retrieved from BEILSTEIN XFIRE, accession no. 4387065 XP002185644 & JOURNAL OF HETEROCYCLIC CHEMISTRY, Nr. 23, - 1986 Seiten 1645-1649,
- DATABASE CROSSFIRE BEILSTEIN [Online] Beilstein Institut zur Förderung der Chemischen Wissenschaften, Frankfurt am Main, DE; retrieved from BEILSTEIN, XFIRE, accession no. 4800778 XP002185645 & JOURNAL OF ORGANIC CHEMISTRY, Bd. 56, Nr. 23, - 1991 Seiten 6508-6516,
- DATABASE CROSSFIRE BEILSTEIN [Online] Beilstein Institut zur Förderung der Chemischen Wissenschaften, Frankfurt aM, DE; retrieved from BEILSTEIN CROSSFIRE, accession no. 608462 XP002185646 & JOURNAL OF THE CHEMICAL SOC. C, - 1971 Seiten 993-999,
- DATABASE BEILSTEIN CROSSFIRE [Online] Beilstein Institut zur Förderung der Chemischen Wissenschaften, Frankfurt aM, DE; retrieved from BEILSTEIN XFIRE, accession no. 2047037 XP002185647 & C.R.HEBD.SEANCES ACAD.SCI., Nr. 203, - 1936 Seite 194
- DATABASE BEILSTEIN CROSSFIRE [Online] Beilstein Institut zur Förderung der Chemischen Wissenschaften, Frankfurt aM, DE; retrieved from BEILSTEIN XFIRE, accession no. 117793 XP002185648 & JOURNAL OF AMER.CHEM.SOC., Nr. 81, - 1959 Seite 4266
- DATABASE BEILSTEIN CROSSFIRE [Online] Beilstein Institut zur Förderung der Chemischen Wissenschaften, Frankfurt aM, DE; retrieved from BEILSTEIN XFIRE, accession no. 4407 XP002185649 & CHEMISCHE BERICHTE, Nr. 31, - 1898 Seite 1857

## Beschreibung

Die vorliegende Erfindung betrifft substituierte und unsubstituierte 3H-Benzo[1,2,3]oxathiazole 2,2-dioxide, 1,3-Dihydro-benzo[1,2,5]thiadiazole 2,2-dioxide und 1,3-Dihydro-benzo[c]isothiazole 2,2-dioxide, ihre Herstellung und Verwendung in Arzneistoffen.

Bekannt sind Aminobenzosultam-Derivate mit Wirkung als Lipoxygensaeinhibitpren (WO 92/05164). Weiterhin bekannt ist die Verwendung entsprechender bifunktioneller Derivate als Ladungstransporter in Photorezeptoren (JP 95/325942). Von Andersen et al. wurde die Synthese von toluolsulfonylgeschützten Derivaten sowie Untersuchungen zu Umsetzungendieser Derivate mit Nukleophilen beschrieben (K. Andersen et al., J. Phys. Org. Chem., 10, 175 - 181 (1997); K. Andersen et al., J Org. Chem., 60, 2003 2007 (1995)).

Die Aufgabe der vorliegenden Erfindung war die Bereitstellung neuer substituierter und unsubstituierter Benzooxathiazole sowie deren Herstellung und Verwendung als pharmazeutische Wirkstoffe. Insbesondere war es Aufgabe, neue substituierte und unsubstituierte Benzooxathiazole zur Behandlung von Diabetes Typ 1 und 2, Insulinresistenz und krankhafter Fettleibigkeit zur Verfügung zu stellen.

Die vorliegende Erfindung betrifft Verbindungen der Formel I 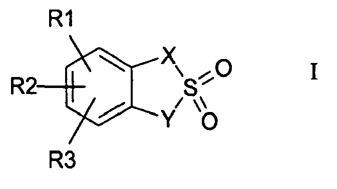 worin bedeuten
- X:: O, N;
- Y:: N;
- R1:: H, F, Cl, Br, J, NH₂, OH, NO₂, COOH; COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
- R2:: H, F, Cl, Br, J, NH₂, OH, NO₂, COOH;
COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
- R3:: COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
und/oder die entsprechenden physiologisch verträglichen Salze.

Die Erfindung betrifft weiterhin bevorzugt Verbindungen der Formel I,
worin bedeuten
- X:: O;
- Y:: N;
- R1:: H, F, Cl, Br, J, NH₂, OH, NO₂, COOH;
COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
- R2:: F, Cl, Br, J, NH₂, OH, NO₂, COOH;
COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können
mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
- R3:: COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann, NCO, NSO₃-(C₁-C₁₀)Alkyl.
und/oder die entsprechenden physiologisch verträglichen Salze.

In einer Verbindung der Formel I können X und Y in bevorzugten Ausführungsformen jeweils unabhängig voneinander die Bedeutung von CH₂, O oder N annehmen.

Die Erfindung bezieht sich auf Verbindungen der Formel I in Form ihrer Racemate, racemischen Mischungen und reinen Enantiomere sowie auf ihre Diastereomere und Mischungen davon.
Die Alkyl-, Alkenyl- und Alkinylreste in den Substituenten R1, R2 oder R3 können sowohl geradkettig wie verzweigt sein.
Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-,
Milch-, Lactobion-, Malein-, Äpfel-, Methansulfon-, Bemstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Salze von chemischen Verbindungen der Formel I können mit Hilfe gebräuchlicher Methoden hergestellt werden, die einem Fachmann auf dem Gebiet geläufig sind. Die Herstellung eines Salzes kann z.B. durch Kombination einer chemischen Verbindung der Formel I mit einer anorganischen oder organischen Säure oder Base in einem Lösungsmittel oder Verdünnungsmittel erfolgen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die am besten geeignete Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardforrnulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

Es wird ein Benzylidendiamin der Formel II, in welchem R1, R2 und R3 die in den vorstehenden Abschnitten definierten Bedeutungen annehmen, mit Sulfondiamin umgesetzt. Insbesondere kann eine chemische Verbindung der Formel 1, in welcher X die Bedeutung N und Y die Bedeutung N annimmt, auf diese Weise hergestellt wird.

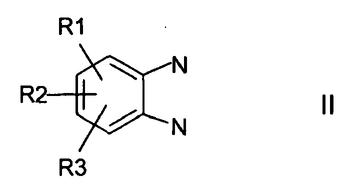

Die Herstellung einer Verbindung der vorliegenden Erfindung ist auch möglich durch Umsetzung eines 2-Aminophenol mit der Formel III, dessen N-Gruppe geschützt ist und dessen Substituenten R1, R2 und R3 die zu Formel I angegebenen Bedeutungen haben mit Sulfurylchlorid und der nachfolgenden Abspaltung der Schutzgruppe. Bevorzugt ist die N-Gruppe des 2-Aminophenols der Formel III durch p-Toluolsulfonyl geschützt. Eine Alternative dieses Verfahrens geht aus von einem 2-Aminophenol der Formel III, worin das 2-Aminophenol ohne Schutzgruppe vorliegt. Dieses 2-Aminophenol der Formel III, dessen N-Gruppe nicht geschützt ist und dessen Substituenten R1, R2 und R3 der in Anspruch 1 jeweils gegebenen Bedeutung entsprechen, wird dabei mit Sulfonyldiimidazol unter basischen Bedingungen umgesetzt. Als Base kann beispielsweise Triethylamin, eine Hünig-Base oder DBU (1,5-Diazabicyclo[4.3.0]non-5-en) verwendet werden.

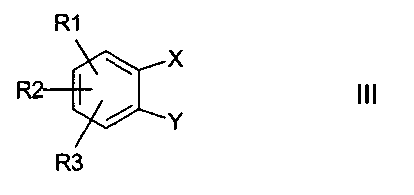

Die Herstellung einer Verbindung der vorliegenden Erfindung ist auch möglich durch ein Verfahren, worin zuerst ein 1-Bromomethyl-2-nitro-benzol der Formel IV, dessen

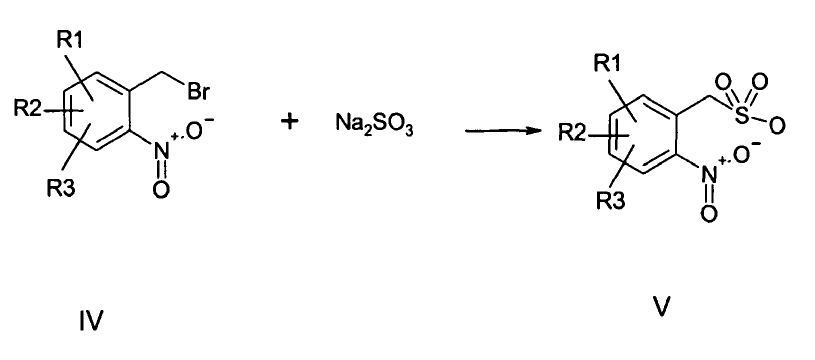

Substituenten R1, R2 und R3 die zu Formel I angegebenen Bedeutungen haben, mit Na₂SO₃ (Natriumsulfit) zu einer Verbindung der Formel V umgesetzt wird, und anschließend die Verbindung mit der Formel V durch Reduktion der Nitrogruppe in das entsprechende Anilin übergeführt wird. Eine Verbindung der Formel I erhält man schließlich durch Erhitzen dieses Anilin der Verbindung mit der Formel V.

Die Erfindung bezieht sich auch auf ein Arzneimittel , welches wenigstens eine der Verbindungen der Formel I und/oder deren physiologisch verträgliche Salze sowie gegebenenfalls zusätzliche Hilfstoffe enthält.

Die Verbindungen der Formel I, und/oder deren physiologisch verträgliche Salze können zur Herstellung von Arzneimitteln verwendet werden.

Solche Arzneimittel eignen sich insbesondere zur Behandlung von Diabetes Typ 1 und 2, Insulinresistenz und krankhafter Dickleibigkeit. Sie eignen sich darüberhinaus auch zur Behandlung von überhöhten Blutfettwerten, Bluthochdruck, Atherosklerose, Fehlfunktionen des Immunsystems, Autoimmunkrankheiten, allergischen Krankheiten wie Asthma, bei Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen, Infektionskrankheiten oder Erkrankungen des Nervensystems wie Alzheimer und Schizophrenie.

Schließlich können Verbindungen der Formel I, und/oder deren physiologisch verträgliche Salze zur Herstellung eines Arzneimittels verwendet werden, wobei dieses Arzneimittel zur Behandlung von Krankheiten insbesondere Diabetes Typ 1 und 2, Insulinresistenz, krankhafter Dickleibigkeit, überhöhten Blutfettwerten, Bluthochdruck, Atherosklerose, Fehlfunktionen des Immunsystems, Autoimmunkrankheiten, allergischen Krankheiten wie Asthma, bei Osteoporose, Proliferationsstörungen wie Krebs und Psoriasis, Krankheiten mit verminderter oder erhöhter Produktion von Wachstumsfaktoren, Hormonen oder Cytokinen, die die Freisetzung von Wachstumshormonen auslösen, Erkrankungen des Nervensystems wie Alzheimer und Schizophrenie und Infektionskrankheiten eingesetzt werden kann.
Die Erfindung betrifft die Herstellung eines Arzneimittels enthaltend wenigstens eine Verbindung dieser Erfindung, wobei der Wirkstoff mit einem pharmazeutisch geeigneten Träger vermischt wird und diese Mischung in eine für die Verabreichung geeignete Form gebracht wird.

### Liste der Abkürzungen:

- aa: Aminosäuren
- DBU: 1,5-Diazabicyclo(4.3.0)non-5-en
- DMSO: Dimethylsulfoxid
- DTT: Dithiotreit
- EDTA: Ethylendiamintetraessigsäure
- EtOAc: Ethyl
- EGTA: Ethytenbis(oxyethylennitrilo)-tetraessigsäure
- h: Stunde
- HPLC: Hochdruckflüssigkeitschromatographie
- MeOH: Methanol
- MOI: mulitplicity of infection
- MS: Massenspektoskopie
- NMR: nuclear magnetic resinance
- PAGE: Polyacrylamidgelelektrophorese
- RT: Raumtemperatur
- RP: reversed phase
- SDS: Natriumdodecylsulfat
- TFA: Trifluoressigsäure

Die nachfolgend aufgeführten Beispiele dienen zur Erläuterung der Erfindung, ohne diese jedoch einzuschränken.

### Beispiele:

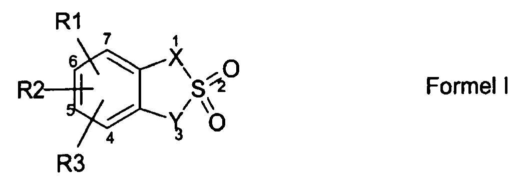

**Tabelle 1**

| Verbindung | R1 | R2 | R3 | X | Y |
|---|---|---|---|---|---|
| | | | | | |
| 2 | 7-H | 6-H | 5-CH₃ | N | O |
| 3 | 7-H | 6-H | 5-H | O | N |
| 4 | An 4 und 5 bilden je zwei Reste R1 und R2 oder R2 und R3 oder R1 und R3 gemeinsam einen anelierten Benzolrest; der jeweils übrige Rest an 7 oder 6 ist H | | | O | N |
| 5 | 7-H | 6-NO₂ | 5-H | O | N |
| 6 | 7-H | 6-NH₂ | 5-H | O | N |
| 7 | 7-H | 6-H | 5-COO(CH₃) | O | N |
| 8 | 7-H | 6-H | 5-COOH | O | N |
| 9 | 7-H | 6-H | 5-CON (CH₂-CH₂-Phenyl-3,4-O-CH₂-Phenyl) | O | N |
| 10 | 7-H | 6-H | 5-CON (CH₂- Phenyl-4-O-(CH₂)₇-CH₃) | O | N |
| 11 | 7-H | 6-H | 5-CON ((CH₂)₁₅-CH₃) | O | N |
| 12 | 7-H | 6-H | 5-COO(CH₃) | N | O |
| 13 | 7-H | 6-H | 5-COOH | N | O |
| 14 | 7-H | 6-H | 5-CON (CH₂-CH₂-Phenyl-3,4-O-CH₂-Phenyl) | N | O |
| 15 | 7-H | 6-H | 5-CON (CH₂-Benzimidazolyl -2,5-S-phenyl) | N | O |

### Hemmstoffe von Phosphatasen sind unter anderem beschrieben in

WO97/3974 (Zimtsäurederivate als Inhibitoren der PTP). Die unspezifische Phosphatasehemmung mittels Vanadiumoxokomplexen und anderen Vanadiumkomplexen führt zur Verbesserung der Insulinresistenz.

### Enzymatische Prüfsysteme zum Nachweis der Hemmung einer Phosphatase

Die Verbindungen der Formel wurden in einem in vitro Assay auf ihre Phosphatase inhibierende Wirkung getestet. Die Enzympräparation und die Durchführung des Assays wurde wie folgt durchgeführt.

### Gewinnung der Enzympräparation:

### A) Zellkultur:

Sf9 Zellen (=Zelltyp von Spodoptera frugiperda; erhältlich bei invitrogen) werden in Spinnerflaschen bei 28°C in Grace's supplementiertem Medium (Gibco-BRL) mit 10% Hitze-inaktiviertem fötalem Kälberserum (Gibco-BRL) gemäß dem Protocol von Summers und Smith (A Manual for Methods for Baculoviruns Vectors and Insect Culture Procedures [Bulletin No. 15555]. Texas A & M University, Texas Agricultural Experiment Station, College Station, TX, 1987) kultiviert.
Konstruktion von rekombinanten Baculovirus Transfervektoren: cDNA kodierend für die regulatorischen and katalytischen Domänen der menschlichen PTP1B, aber ohne die carboxy-terminale hydrophobe Region (entsprechend 1-299 aa) wurde über Polymerasekettenreaktion über Primer mit angefügten Klonierungsstellen und geeigneten cDNA Matrizen (erhältlich beispielsweise von invitrogen) erhalten und dann in
Baculovirusexpressionvektoren (Amersham Pharmacia Biotech.) kloniert. Die rekombinanten Baculoviren wurden mit Hilfe des Bac-to-Bac Baculovirus Expressionsystems (erhältlich von Gibco-BRL) hergestellt. Das Gen wurde in das pFASTBAC Donorplasmid kloniert (erhältlich von Life Technologies). Das resultierende Plasmid wurde in kompetente DH10BAC Escherichia coli Zellen (erhältlich von Life Technologies) transformiert. Nach der Transposition und Antibiotikaselektion wurde die rekombinante Plasmid-DNA von selektierten E. coli Kolonien isoliert und dann für die Transfektion von Sf9 Insektenzellen benutzt. Der Viruspartikel im Überstandsmedium wurde dreimal amplifiziert bis auf ein virales Stockvolumen von 500 ml.

### B) Produktion von rekombinantem Protein:

Baculovirusinfection einer 500-ml Spinnerkultur von Sf9 Zellen wurde im wesentlichen durchgeführt wie von Summers und Smith beschrieben (s.o.). Sf9 Zellen bei einer Dichte von 1-3 x 10⁶ Zellen/ml wurden durch Zentrifugation bei 300 g für 5 min pelletiert, der Überstand wurde entfernt und die Zellen in einer Dichte von 1x10⁷ Zellen/ml in einem geeigneten rekombinanten Viralstock (MOI 10) resuspendiert. Nach vorsichtigem Schütteln für 1.5 Std. bei Raumtemperatur wurde frisches Medium hinzugegeben, um eine Zelldichte von 1x10⁶ Zellen/ml zu erreichen. Die Zellen wurden dann in Suspension bei 28°C für geeignete Perioden nach Postinfektion kultiviert.

### C) Zelluläre Fraktionierung und Gesamtzellextrakte von infizierten Sf9 Zellen:

Nach der Postinfektion wurden Aliquots einer Analyse der Proteinexpression durch SDS-PAGE und Westernblotanalyse unterzogen. Die zelluläre Fraktionierung wurde durchgeführt wie beschrieben (Cromlish, W. and Kennedy, B. Biochem. Pharmacol. 52: 1777-1785, 1996). Gesamtzellextrakte wurden von 1-ml Aliquots der infizierten Sf9 Zellen nach bestimmten Zeiten Postinfektion gewonnen. Die pelletierten Zellen (300xg, 5 min) wurden einmal in Phosphate-gepufferter Saline (4°C) gewaschen, resuspendiert in 50 µl Wasser und durch wiederholtes Einfrieren/Auftauen aufgeschlossen. Proteinkonzentrationen wurden mit Hilfe der Bradfordmethode und Rinderserumalbumin als Standard bestimmt.

### Durchführung des Assays:

### A) Dephosphorylierung eines Phosphopeptids:

Dieser Assay beruht auf der Freisetzung von Phosphat aus einem Konsensussubstratpeptid, welches im nanomolaren Konzentrationsbereich durch die Malachitgrün-Ammoniummolybdate-Methode (Lanzetta, P.A., Alvarez, L.J., Reinach, P.S., Candia, O.A. Anal Biochem. 100: 95-97, 1979) adaptiert für das Mikrotiterplattenformat nachgewiesen wird. Das Dodecatrisphosphopeptid, TRDIYETDYYRK (Biotrend, Köln) entpricht den Aminosäuren 1142-1153 der katalytischen Domaäne des Insulinrezeptors und wird (auto)phosphoryliert an den Tyrosinresten 1146, 1150, und 1151. Die rekombinante hPTP1B wurde mit Assaypuffer verdünnt (40 mM Tris/HCl, pH 7.4, 1 mM EDTA, 20 mM DTT), entsprechend einer Aktivität von 1000-1500 nmoi/min/mg Protein und (eine 20 µl-Portion) dann vorinkubiert (15 min, 30°C) in Ab- oder Anwesenheit der Testsubstanz (5 µl) in der gewünschten Konzentration (Endkonz. DMSO 2 % max.) in einem Gesamtvolumen von 90 µl (Assaypuffer). Zum Start der Dephosphorylierungsreaktion wurde das Peptidsubstrat (10 µl, vorgewärmt auf 30°C) zur vorinkubierten Enzympräparation mit oder ohne Testsubstanz (Endkonz. 0.2-200 µM) hinzugegeben und die Inkubation für 1 Std. fortgesetzt. Die Reaktion wurde beendet durch Hinzufügen von 100 µl Malachitgrünhydrochlorid (0.45 %, 3 Teile), Ammoniummolybdattetrahydrat (4.2 % in 4 N HCl, 1 Teil) und 0.5 % Tween 20 als Stoplösung. Nach 30 min Inkubation bei 22°C für die Entwicklung der Farbe wurde die Absorption bei 650 nm mit Hilfe eines Mikrotiterplattenlesegeräts (Molecular Devices) bestimmt. Proben und Leerwerte wurden als Dreifachwerte gemessen. Die PTP1 B Aktivität wurde als Nanomole an freigesetztem Phosphat pro min und mg Protein mit Kaliumphosphat als Standard berechnet. Die Inhibition der rekombinanten hPTP1 B durch Testsubstanzen wurde als Prozent der Phosphatasekontrolle berechnet. Die IC₅₀-Werte zeigen signifikante Übereinstimmung mit einer Vier-Parameter-nichtlinearen logistischen Regressionskurve.

### B) Spaltung von p-Nitrophenylphosphat:

Dieser Assay beruht auf der Absorptionsveränderung des nicht-physiologischen Substrats p-Nitrophenylphosphat während der Spaltung zu Nitrophenol unter Standardbedingungen (Tonks, N.K., Diltz, C.D:, Fischer, E.H. J. Biol. Chem. 263: 6731-6737, 1988; Burke T.R., Ye, B., Yan, X.J., Wang, S.M., Jia, Z.C., Chen, L., Zhang, Z.Y., Barford, D. Biochemistry 35: 15989-15996, 1996). Die Inhibitoren werden in geeigneter Verdünnung zu den Reaktionsgemischen pipettiert, die 0.5-5 mM p-Nitrophenylphosphat enthalten. Die folgenden Puffer wurden benutzt (Gesamtvolumen 100 µl): (a) 100 mM Natriumazetat (pH 5.5), 50 mM NaCl, 0.1 % (w/v) Rinderserumalbumin, 5 mM Glutathion, 5 mM DTT, 0.4 mM EGTA und 1 mM EDTA; (b) 50 mM Hepes/KOH (pH 7.4), 100 mM NaCl, 0.1 % (w/v) Rinderserumalbumin, 5 mM Glutathion, 5 mM DTT und 1 mM EDTA. Die Reaktion wurde gestartet durch Zugabe von Enzym und in Mikrotiterplatten bei 25°C für 1 Std. durchgeführt. Die Reaktion wurde beendet durch Zugabe 100 µl 0.2 N NaOH. Die Enzymaktivität wurde bestimmt durch Messung der Absorption bei 405 nm mit geeigneten Korrekturen für Absorption der Testsubstanzen und von p-Nitrophenylphosphat. Die Ergebnisse wurden als Prozent der Kontrolle ausgedrückt, in dem die Menge an gebildetem p-Nitrophenol in den Testsubstanz-behandelten Proben (nmol/min/mg Protein) mit der Menge in den unbehandelten Proben verglichen wurde. Der Mittelwert und die Standardabweichung wurden berechnet, die IC50-Werte wurden durch Regressionsanalyse des linearen Anteils der Hemmkurven bestimmt.

Aus den Testergebnissen geht hervor, daß die erfindungsgemäßen Verbindungen der Formel I eine hemmende Wirkung auf die Phosphotyrosinphosphatase 1B (PTP1B) aufweisen. Es ist bekannt, daß die PTP1B eine wichtige Funktion bei intrazellulären Signalkaskaden ausübt. Die Verbindungen sind deshalb geeignet zur Behandlung insbesondere von Diabetes Typ 1 und 2, Insulinresistenz und krankhafter Fettleibigkeit. Die Verbindungen sind wegen ihrer Hemmung der PTP1B auch geeignet zur Behandlung von Hyperglycerimia, Bluthochdruck, Atherosklerose, Fehlfunktionen des Immunsystems, Autoimmunkrankheiten, Allergische Krankheiten wie Asthma, bei Osteoporose, Proliferationsstörungen wie Krebs und Psoriasis, Krankheiten mit verminderter oder erhöhter Produktion von Wachstumsfaktoren, Hormonen oder Cytokinen die die Freisetzung von Wachstumshormonen auslösen, Erkrankungen des Nervensystems wie Alzheimer und Schizophrenie und Infektionskrankheiten.
Herstellung von beispielhaften Verbindungen (Nummerierung entsprechend zu Tabelle 1):
Nachfolgend wird die Herstellung einiger Verbindungen detailliert beschrieben, die übrigen Verbindungen der Formel I wurden analog erhalten:

### Verbindung 2: 6-Methyl-3H-benzo[1,2,3]oxathiazole 2,2-dioxid

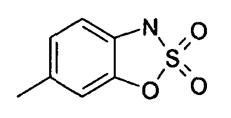

### N-(2-Hydroxy-4-methyl-phenyl)-4-methyl-benzosulfonamid

Zu einer Lösung von 2-Amino-5-methyl-phenol (1.23 g, 10 mmol) in CH₂Cl₂ (20 mL) gibt man Pyridin (810 µL) und anschließend portionsweise p-Toluolsulfonsäurechlorid (1.91 g, 10 mmol). Das Reaktionsgemisch wird 4 h bei 40° C gerührt. Das Lösungsmittel wird im Vakuum abdestilliert, der Rückstand mit Ethylcetat versetzt und der Feststoff abgesaugt.
Ausbeute: 2.32 g (83%)

### 6-Methyl-3-(toluol-4-sulfonyl)-3H-benzo[1,2,3]oxathiazol 2,2-dioxid

Zu einer Lösung von N-(2-Hydroxy-4-methyl-phenyl)-4-methyl-benzosulfonamid (1.5 g, 5.41 mmol) und Triethylamin (1.5 mL) tropft man bei -78° C langsam eine Lösung von Sulfurylchlorid (450 µL, 5.41 mmol) in CH₂Cl₂ (10 mL) und rührt eine Stunde bei -78°C. Nach Auftauen auf RT wird das Lösungsmittel im Vakuum abdestilliert und der Rückstand durch RP-Chromatographie gereinigt.
Ausbeute: 592 mg (32%).

### 6-Methyl-3H-benzo[1,2,3]oxathiazole 2,2-dioxid

6-Methyl-3-(toluol-4-sulfonyl)-3H-benzo[1,2,3]oxathiazol 2,2-dioxid (100 mg, 0.295 mmol) wird in Acetonitril (5 mL) gelöst. Zu dieser Lösung wird eine Natriumazidlösung (aus 20 mg, 0.29 mmol Natriumazid in 1 mL H₂O) zugegeben und über Nacht bei RT gerührt. Anschließend wird noch 1 h bei 60° C gerührt, das Lösungsmittel im Vakuum abdestilliert und der Rückstand durch RP-Chromatographie gereinigt.
Ausbeute: 47 mg (85%).
¹H-NMR (D₆-DMSO): δ 6.51 (d, 1 H, aryl), 6.45 (d, 1 H, aryl) 6.29 (m, 1 H, aryl), 2.14 (s, 3 H, CH₃).
MS (ESI-MS, ES-) 184.9 (M-1).

### Verbindung 3: 5-Methyl-3H-benzo[1,2,3]oxathiazole 2,2-dioxide

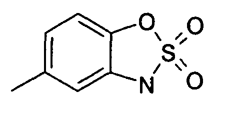

5-Methyl-3H-benzo[1,2,3]oxathiazole 2,2-dioxide wurde entsprechend der unter Beispiel 2 beschriebenen Sequenz ausgehend von 2-Amino-4-methyl-phenol synthetisiert.
¹H-NMR (D₆-DMSO): δ 6.53 (d, 1 H, aryl), 6.23 (m, 1 H,aryl), 6.10 (dd, 1 H, aryl), 2.13 (s, 3 H, CH₃).
MS (ESI-MS, ES-) 184.9 (M-1).

### Verbindung 4: 1 H-3-Oxa-2-thia-1-aza-cyclopenta[a]naphthyl 2,2-dioxid

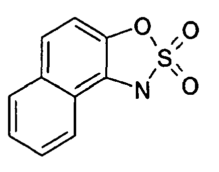

1 H-3-Oxa-2-thia-1-aza-cyclopenta[a]naphthyl 2,2-dioxid wurde entsprechend der unter Beispiel 2 beschriebenen Sequenz ausgehend von 1-Amino-naphthyl-2-ol synthetisiert.
¹H-NMR (D₆-DMSO): δ 7.8 (dd, 1 H, aryl), 7.66 (dd, 1 H, aryl), 7.23 (m, 2 H, aryl) 7.1 (d, 1 H aryl), 6.9 (d, 1 H, aryl).
MS (ESI-MS, ES-) 221 (M-1).

### Verbindung 5: 6-Nitro-3H-benzo[1,2,3]oxathiazol 2,2-dioxid

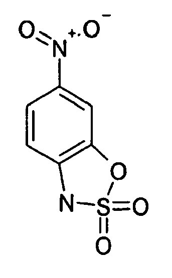

Eine Lösung von 2-Amino-5-nitro-phenol (7.7 g, 50 mmol) in Acetonitril (300 mL) wird mit N-Ethyldiisopropylamin (18.7 mL, 110 mmol) und N,N'-Sulfuryldiimidazol (10.8 g, 55 mmol) versetzt und 18 h unter RF gekocht. Nach Abkülen auf RT destilliert man das Lösungsmittel im Vakuum ab, nimmt den Rückstand in 1 n HCL auf und extrahiert das Produkt mit Ethylacetat. Das Produkt wird anschließend durch Flash Chromatographie (17:2:1, EtOAc/MeOH/H₂O) gereinigt.
Ausbeute: 8.3 g (76.9%).
¹H-NMR (D₆-DMSO): δ 7.6 (dd, 1 H, aryl), 7.58 (s, 1 H, aryl), 6.55 (d, 1 H, aryl).
MS (ESI-MS, ES-) 214.9 (M-1).

### Verbindung 6: 6-Amino-3H-benzo[1,2,3]oxathiazol 2,2-dioxid

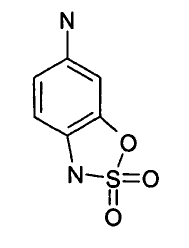

Eine Lösung von 6-Nitro-3H-benzo[1,2,3]oxathiazol 2,2-dioxid (Beispiel 5) (8.1 g, 37 mmol) in Methanol (250 mL) wird in Anwesenheit von Pd-C unter Normaldruck hydriert. Anschließend wird der Katalysator abfiltriert, die klare Lösung mit methanolischer
HCL (1 N) versetzt und das Lösungsmittel im Vakuum abdestilliert. Der Rückstand wird in Ethanol gelöst und das Produkt nach Zugabe von Diethylether kristallisiert. 3.95 g (57.3 %).

### Verbindung 7: 2,2-Dioxo-2,3-dihydro-2,6-benzo[1,2,3]oxathiazol-5-carbonsäuremethylester

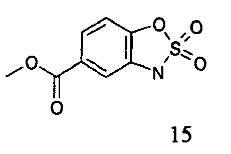

3g 3-Amino-4-hydroxy-benzoesäuremethylester (0,018 Mol), 3,9 g Sulfonyldiimidazol (0,02 Mol) und 3g DBU (0,02 Mol) werden in 50 ml Acetonitril gelöst, die Lösung entgast und anschließend 3h zur Siedetemperatur erhitzt. Zur Aufarbeitung wird die Lösung mit 120 ml Ethylacetat verdünnt und mit 50 ml 1N HCl extrahiert. Die organische Phase wird über Magnesiumsulfat getrocknet und das Lösungsmittel unter vermindertem Druck abdestilliert.
Das Rohprodukt wird ohne Reinigung für die nächste Stufe eingesetzt.
Ausbeute: 3,6 g (88%)
¹H-NMR (D6-DMSO): δ 7.55 (dd, 1 H, aromat.), 7.45 (d, 1 H, aromat.), 7.23 (d, 1 H, aromat.), 3.85 (s, 3H, OMe). MS (ESI-MS) 230.1 (M+1).

### Verbindung 8: 2,2-Dioxo-2,3-dihydro-2,6-benzo[1,2,3]oxathiazol-5-carbonsäure

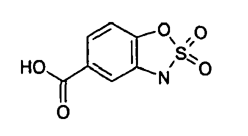

3,3 g 1 (21 mmol) werden in einer Lösung aus 1,25 NaOH in 70 ml Wasser gelöst. Das Reaktionsgemisch wird 4h bei 25°C gerührt.
Anschließend wird mit 2N HCl auf pH 2 angesäuert und zur Trockene eingedampft. Zum Abtrennen des NaCl wird der Rückstand in 150 ml Aceton aufgenommen, filtriert und das Lösungsmittel abdestilliert. Das Rohprodukt wird ohne Reinigung für die nächste Stufe eingesetzt.
Ausbeute: 2,3 g (75%)
¹H-NMR (D6-DMSO): δ 7.54 (dd, 1 H, aromat.), 7.40 (d, 1 H, aromat.), 7.27 (d, 1 H, aromat.). MS (ESI-MS) 216.1 (M+1).

### Verbindung 9: 2,2-Dioxo-2,3-dihydro-2,6-benzo[1,2,3]oxathiazol-5-carbonsäure [2-(3,4-bis- benzyloxy-phenyl)-ethyl]-amid

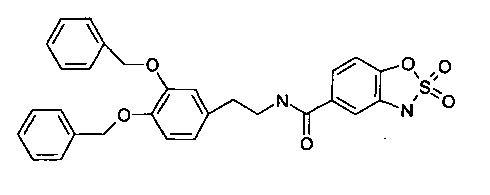

Eine Lösung von 100 mg 3 (0,46 mmol), 160 mg 2-(3,4-Bis-benzyloxy-phenyl)-ethylamine Hydrochlorid (0,6 mmol), 115 mg EDC (0,6 mmol), 81 mg HOBT und 260 mg Ethyldiisopropylamin in 2 ml DMF wird 5h bei 25°C gerührt. Anschließend wird mit 20 ml Ethylacetat verdünnt und mit 10 ml 2N HCl extrahiert. Die organische Phase wird über Magnesiumsulfat getrocknet und das Lösungsmittel abdestilliert. Das Rohprodukt wird mittels HPLC (RP18, Acetonitril/Wasser 0,1% TFA)
gereinigt.
Ausbeute: 66 mg (40%).
¹H-NMR (D6-DMSO): δ 8.49 (t, 1 H, NH), 7.46-7.23 (m, 13 H, aromat.), 6.97 (d, 2 H, aromat.), 6.74 (dd, 1 H, aromat.), 5.08 (s, 4H, OCH₂), 3.43 (dt, 2H, NCH₂), 2.74 (2H, t, CH₂). MS (ESI-MS) 531.2 (M+1).

### Verbindung 10: 2,2-Dioxo-2,3-dihydro-2,6-benzo[1,2,3]oxathiazol-5-carbonsäure 4-octyloxy- benzylamid 4

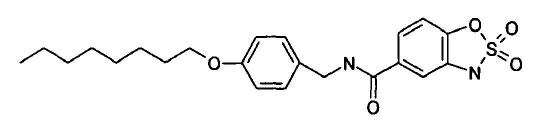

Verbindung 4 wird wie für Verbindung 3 beschrieben dargestellt.
Ausbeute: 63 mg (52%)
¹H-NMR (D6-DMSO): δ 8.95 (t, 1 H, NH), 7.52 (dd, 1 H, aromat.), 7.45 (d, 1 H, aromat.), 7.3 (d, 1 H, aromat.), 7.22 (d, 2 H, aromat.), 6.86 (d, 2 H, aromat.), 4.37 (d, 2H, NCH₂), 3.91 (t, 2H, OCH₂), 1.65 (m, 2H, CH₂), 1.45-1.2 (m, 10H, CH₂), 0.86 (t, 3H, CH₃). MS (ESI-MS) 433.2 (M+1).

### Verbindung 11: 2,2-Dioxo-2,3-dihydro-2,6-benzo[1,2,3]oxathiazol-5-carbonsäurehexadecylamid 5

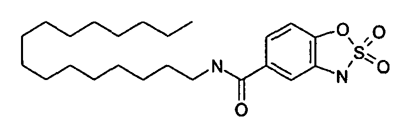

Verbindung 5 wird wie für Verbindung 3 beschrieben dargestellt.
Ausbeute: 51 mg (26%)
¹H-NMR (D6-DMSO): δ 8.35 (t, 1 H, NH), 7.38 (d, 1 H, aromat.), 7.34 (s, 1 H, aromat.), 7.2 (d, 1 H, aromat.), 3.21 (dt, 2H, NCH₂), 1.5 (m, 2H, CH₂), 1.4 (m, 26H, CH₂), 0.85 (t, 3H, CH₃). MS (ESI-MS) 439.3 (M+1).

### Verbindung 12: 2,2-Dioxo-2,3-dihydro-2,6-benzo[1,2,3]oxathiazol-6-carbonsäuremethylester 6

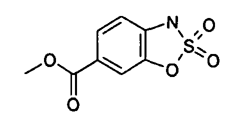

Verbindung 6 wird wie für Verbindung 1 beschrieben dargestellt.
Ausbeute: 3,35 g (82%)
¹H-NMR (D6-DMSO): δ 7.43 (dd, 1 H, aromat.), 7.19 (d, 1 H, aromat.), 6.53 (d, 1 H, aromat.). MS (ESI-MS) 227.9 (M-1).

### Verbindung 13: 2,2-Dioxo-2,3-dihydro-2,6-benzo[1,2,3]oxathiazol-6-carbonsäure 7

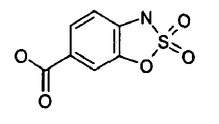

Verbindung 7 wird wie für Verbindung 2 beschrieben aus 6 dargestellt.
Ausbeute: 2,2 g (71%)
¹H-NMR (D6-DMSO): δ 7.46 (dd, 1 H, aromat.), 7.25 (d, 1 H, aromat.), 6.46 (d, 1 H, aromat.) 3.73 (s, 3H, Ome). MS (ESI-MS) 213.9 (M-1).

### Verbindung 14:2,2-Dioxo-2,3-dihydro-2,6-benzo[1,2,3]oxathiazol-6-carbonsäure [2-(3,4-bis- benzyloxy-phenyl)-ethyl]-amid 8

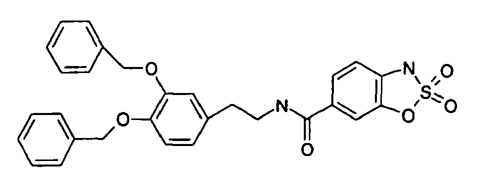

Verbindung 8 wird wie für Verbindung 3 beschrieben aus 7 dargestellt.
Ausbeute: 41 mg g (25%)
¹H-NMR (D6-DMSO): δ 8.33 (t, 1H, NH), 7.55 (m, 2 H, aromat.), 7.45-7.3 (m, 10 H, aromat.), 6.96 (d, 2 H, aromat.), 6.86 (d, 1 H, aromat.), 6.74 (dd, 1 H, aromat.), 5.08 (s, 4H, OCH₂), 3.41 (dt, 2H, NCH₂), 2.73 (2H, t, CH₂). MS (ESI-MS) 531.3 (M+1).

### Verbindung 15: 2,2-Dioxo-2,3-dihydro-2,6-benzo[1,2,3]oxathiazol-6-carbonsäure (5-phenylsulfanyl- 1 H-benzoimidazol-2-ylmethyl)-amid 9

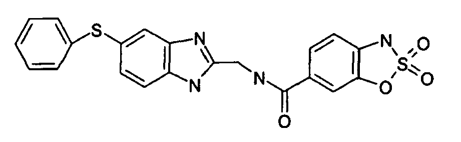

Verbindung 9 wird wie für Verbindung 3 beschrieben aus 7 dargestellt.
Ausbeute: 69 mg g (34%)
¹H-NMR (D6-DMSO): δ 8.96 (t, 1 H, NH), 7.75 (d, 1 H, aromat.), 7.61 (d, 1H, aromat.), 7.46, (m, 2H, aromat.) 7.41-7.3 (m, 6 H, aromat.), 6.55 (d, 1 H, aromat.), 4.81 (d, 2H, NCH₂). MS (ESI-MS) 453.2 (M+1).

## Patentansprüche

1. Verbindungen der Formel I 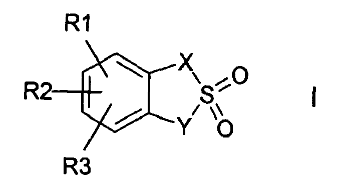 worin bedeuten
X: O, N;
Y: N;
R1: H, F, Cl, Br, J, NH₂, OH, NO₂, COOH; COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂,
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
R2: H, F, Cl, Br, J, NH₂, OH, NO₂, COOH; COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann, NCO, NSO₃-(C₁-C₁₀)Alkyl;
R3: COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann, NCO, NSO₃-(C₁-C₁₀)Alkyl;
und/oder die entsprechenden physiologisch verträglichen Salze.

2. Verbindungen der Formel nach Anspruch 1, **dadurch gekennzeichnet, daß** darin bedeuten
X: O;
Y: N;
R1: H, F, Cl, Br, J, NH₂, OH, NO₂, COOH; COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann, NCO, NSO₃-(C₁-C₁₀)Alkyl;
R2: F, Cl, Br, J, NH₂, OH, NO₂, COOH; COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂:
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann,
NCO, NSO₃-(C₁-C₁₀)Alkyl;
R3: COO(C₁-C₆)Alkyl, CONH₂, CONH(C₁-C₁₆)Alkyl, CONH(C₁-C₁₆)Alkenyl, CONH(C₁-C₆)Alkyl-Phenyl, wobei Phenyl ein bis dreifach substituiert sein kann mit O-(C₁-C₁₀)Alkyl oder O-(C₁-C₁₀)Alkyl-Phenyl, CONH(C₁-C₆)Alkyl-Benzimidazol, wobei der Benzimidazolring ein- bis dreifach substituiert sein kann mit S-Phenyl, welches wiederum ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
O-(C₁-C₆)Alkyl;
(C₁-C₆)Alkyl, (C₂-C₆)Alkenyl, (C₂-C₆)Alkinyl, (C₁-C₆)Alkyl-COOH, (C₁-C₆)Alkyl-Aryl, wobei Aryl gleich Phenyl, Naphthyl, Biphenyl, Thienyl oder Pyridyl sein kann und der Arylteil jeweils ein- bis dreifach substituiert sein kann mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
Phenyl, Biphenyl, 1- oder 2-Naphthyl, 2-,3- oder 4-Pyridyl, 2- oder 3-Furanyl oder 2- oder 3-Thienyl, wobei die Phenyl-, Biphenyl-, Naphthyl-, Pyridyl-, Furanyl- oder Thienylringe jeweils ein- bis dreifach substituiert sein können mit F, Cl, Br, J, OH, CF₃, NO₂, CN, OCF₃, O-(C₁-C₁₀)Alkyl, NH₂, NH(C₁-C₆)Alkyl, COOH, COO(C₁-C₆)Alkyl, CONH₂;
(C₃-C₁₈)Cycloalkyl, wobei in den Alkylresten ein oder mehrere Wasserstoff(e) durch Fluor ersetzt sein können oder ein Wasserstoff durch OH, (C₁-C₆)Alkyl-Phenyl oder O-(C₁-C₆)Alkyl-Phenyl ersetzt sein kann, NCO, NsO₃-(C₁-C₁₀)Alkyl;
und/oder die entsprechenden physiologisch verträglichen.

3. Arzneimittel enthaltend wenigstens eine Verbindung gemäß einem oder mehreren der Ansprüche 1 und 2.

4. Verwendung einer Verbindung gemäß einem oder mehreren der Ansprüche 1 und 2 zur Herstellung eines Arzneimittels.

5. Verwendung einer Verbindung gemäß einem oder mehreren der Ansprüche 1 und 2 zur Herstellung eines Arzneimittels zur Behandlung von Diabetes Typ 1.

6. Verwendung einer Verbindung gemäß einem oder mehreren der Ansprüche 1 und 2 zur Herstellung eines Arzneimittels zur Behandlung von Diabetes Typ 2.

7. Verwendung einer Verbindung gemäß einem oder mehreren der Ansprüche 1 und 2 zur Herstellung eines Arzneimittels zur Behandlung von Insulinresistenz.

8. Verwendung einer Verbindung gemäß einem oder mehreren der Ansprüche 1 und 2 zur Herstellung eines Arzneimittels zur Behandlung von krankhafter Fettleibigkeit.

9. Verfahren zur Herstellung eines Arzneimittels enthaltend eine oder mehrere der Verbindungen gemäß einem oder mehreren der Ansprüche 1 und 2, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula I 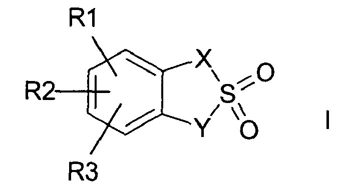 in which
X is O, N;
Y is N;
R1 is H, F, Cl, Br, I, NH₂, OH, NO₂, COOH;
COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cycloalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
R2 is H, F, Cl, Br, I, NH₂, OH, NO₂, COOH; COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cycloalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
R3 is COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cycloalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
and/or the corresponding physiologically acceptable salts.

2. A compound of the formula I as claimed in claim 1, wherein
X is O;
Y is N;
R1 is H, F, Cl, Br, I, NH₂, OH, NO₂, COOH; COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cycloalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
R2 is F, Cl, Br, I, NH₂, OH, NO₂, COOH;
COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or 3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cydoalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
R3 is COO(C₁-C₆)alkyl, CONH₂, CONH(C₁-C₁₆)alkyl, CONH(C₁-C₁₆)alkenyl, CONH(C₁-C₆)alkyl-phenyl, where phenyl may be mono- to trisubstituted by O-(C₁-C₁₀)alkyl or O-(C₁-C₁₀)alkyl-phenyl, CONH(C₁-C₆)alkyl-benzimidazole, where the benzimidazole ring may be mono- to trisubstituted by S-phenyl, which for its part may be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
O-(C₁-C₆)alkyl;
(C₁-C₆)alkyl, (C₂-C₆)alkenyl, (C₂-C₆)alkynyl, (C₁-C₆)alkyl-COOH, (C₁-C₆)alkyl-aryl, where aryl may be phenyl, naphthyl, biphenyl, thienyl or pyridyl and the aryl moiety may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
Phenyl, biphenyl, 1- or 2-naphthyl, 2-, 3- or 4-pyridyl, 2- or 3-furanyl or 2- or
3-thienyl, where the phenyl, biphenyl, naphthyl, pyridyl, furanyl or thienyl rings may in each case be mono- to trisubstituted by F, Cl, Br, I, OH, CF₃, NO₂, CN, OCF₃, O-(C₁-C₁₀)alkyl, NH₂, NH(C₁-C₆)alkyl, COOH, COO(C₁-C₆)alkyl, CONH₂;
(C₃-C₁₈)cycloalkyl, where in the alkyl radicals one or more hydrogens may be replaced by fluorine or one hydrogen may be replaced by OH, (C₁-C₆)alkyl-phenyl or O-(C₁-C₆)alkyl-phenyl,
NCO, NSO₃-(C₁-C₁₀)alkyl;
and/or the corresponding physiologically acceptable salts.

3. A medicament, comprising at least one compound as claimed in one or more of claims 1 and 2.

4. The use of a compound as claimed in one or more of claims 1 and 2 for preparing a medicament.

5. The use of a compound as claimed in one or more of claims 1 and 2 for preparing a medicament for the treatment of type 1 diabetes.

6. The use of a compound as claimed in one or more of claims 1 and 2 for preparing a medicament for the treatment of type 2 diabetes.

7. The use of a compound as claimed in one or more of claims 1 and 2 for preparing a medicament for the treatment of insulin resistance.

8. The use of a compound as claimed in one or more of claims 1 and 2 for preparing a medicament for the treatment of pathological obesity.

9. A process for preparing a medicament comprising one or more compounds as claimed in one or more of claims 1 and 2, which comprises mixing the active compound with a pharmaceutically acceptable excipient and bringing this mixture into a form suitable for administration.

## Revendications

1. Composés de formule I 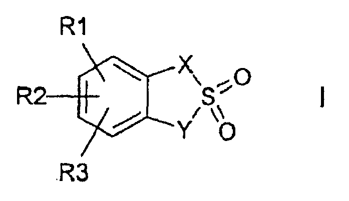 dans laquelle
X représente O, N ;
Y représente N ;
R1 représente H, F, Cl, Br, I, NH₂, OH, NO₂, COOH ;
un groupe COO-alkyle(C₁-C₆), CONH₂, CONH-alkyle(C₁-C₁₆), CONH-alcényle(C₁-C₁₆), CONH-alkyl (C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle(C₁-C₁₀), NH₂, NH-alkyle (C₁-C₆), COOH, COO-alkyle(C₁-C₆), CONH₂ ;
un groupe O-alkyle(C₁-C₆) ;
un groupe alkyle en C₁-C₆, alcényle en C₂-C₆, alcynyle en C₂-C₆, alkyl (C₁-C₆) -COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
R2 représente H, F, Cl, Br, I, NH₂, OH, NO₂, COOH ;
un groupe COO-alkyle(C₁-C₆), CONH₂,
CONH-alkyle(C₁-C₁₆), CONH-alcényle(C₁-C₁₆), CONH-alkyl(C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe O-alkyle(C₁-C₆) ;
un groupe alkyle en C₁-C₆, alcényle en C₂-C₆, alcynyle en C₂-C₆, alkyl (C₁-C₆) -COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle (C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
R3 représente un groupe COO-alkyle(C₁-C₆), CONH₂, CONH-alkyle (C₁-C₁₆), CONH-alcényle(C₁-C₁₆), CONH-alkyl(C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle (C₁-C₁₀), NH₂, NH-alkyle (C₁-C₆), COOH, COO-alkyle(C₁-C₆), CONH₂ ; un groupe O-alkyle(C₁-C₆) ;
un groupe alkyle en C₁-C₆, alcényle en C₂-C₆, alcynyle en C₂-C₆, alkyl(C₁-C₆)-COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle (C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
et/ou sels physiologiquement acceptables correspondants.

2. Composés de formule I selon la revendication 1, **caractérisés en ce que** dans cette formule
X représente O ;
Y représente N ;
R1 représente H, F, Cl, Br, I, NH₂, OH, NO₂, COOH ;
un groupe COO-alkyle(C₁-C₆), CONH₂, CONH-alkyle(C₁-C₁₆), CONH-alcényle(C₁-C₁₆), CONH-alkyl(C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle(C₁-C₁₀), NH₂, NH-alkyle (C₁-C₆), COOH, COO-alkyle(C₁-C₆), CONH₂ ;
un groupe O-alkyle (C₁-C₆) ;
un groupe alkyle en C₁-C₆, alcényle en C₂-C₆, alcynyle en C₂-C₆, alkyl (C₁-C₆) -COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
R2 représente F, Cl, Br, I, NH₂, OH, NO₂, COOH ;
un groupe COO-alkyle(C₁-C₆), CONH₂, CONH-alkyle(C₁-C₁₆), CONH-alcényle(C₁-C₁₆), CONH-alkyl(C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe O-alkyle(C₁-C₆) ;
un groupe alkyle en C₁-C₆, alcényle en C₂-C₆, alcynyle en C₂-C₆, alkyl(C₁-C₆)-COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
R3 représente un groupe COO-alkyle(C₁-C₆), CONH₂, CONH-alkyle (C₁-C₁₆), CONH-alcényle(C₁-C₁₆), CONH-alkyl(C₁-C₆)-phényle, le radical phényle pouvant être une à trois fois substitué par O-alkyle(C₁-C₁₀) ou O-alkyl(C₁-C₁₀)-phényle, un groupe CONH-alkyl(C₁-C₆)-benzimidazole, le cycle benzimidazole pouvant être une à trois fois substitué par un groupe S-phényle qui peut être à son tour une à trois fois substitué par F, Cl, Br, I, OH, CF₃, NO₂, CN, OCF₃, O-alkyle(C₁-C₁₀), NH₂, NH-alkyle (C₁-C₆), COOH, COO-alkyle(C₁-C₆), CONH₂ ;
un groupe O-alkyle(C₁-C₆) ;
un groupe alkyle en C₁-C₆, alcényle en C₂-C₆, alcynyle en C₂-C₆, alkyl (C₁-C₆)-COOH, alkyl(C₁-C₆)-aryle, le fragment aryle pouvant être phényle, naphtyle, biphényle, thiényle ou pyridyle et le fragment aryle pouvant être chaque fois substitué une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un cycle phényle, biphényle, 1- ou 2-naphtyle, 2-, 3- ou 4-pyridyle, 2- ou 3-furannyle ou 2- ou 3-thiényle, les cycles phényle, biphényle, naphtyle, pyridyle, furannyle et thiényle pouvant être substitués chacun une à trois fois par F, Cl, Br, I, OH, CF₃, NO₂, CN, OCF₃, O-alkyle(C₁-C₁₀), NH₂, NH-alkyle(C₁-C₆), COOH, COO-alkyle(C₁-C₆), CONH₂ ;
un groupe cycloalkyle en C₃-C₁₈, un ou plusieurs atomes d'hydrogène dans les radicaux alkyle pouvant être remplacé(s) par le fluor ou un atome d'hydrogène pouvant être remplacé par OH ou par un groupe alkyl(C₁-C₆)-phényle ou O-alkyl(C₁-C₆)-phényle ;
un groupe NCO, NSO₃-alkyle(C₁-C₁₀) ;
et/ou sels physiologiquement acceptables correspondants.

3. Médicament contenant au moins un composé selon une ou plusieurs des revendications 1 et 2.

4. Utilisation d'un composé selon une ou plusieurs des revendications 1 et 2, pour la fabrication d'un médicament.

5. Utilisation d'un composé selon une ou plusieurs des revendications 1 et 2, pour la fabrication d'un médicament destiné au traitement du diabète de type 1.

6. Utilisation d'un composé selon une ou plusieurs des revendications 1 et 2, pour la fabrication d'un médicament destiné au traitement du diabète de type 2.

7. Utilisation d'un composé selon une ou plusieurs des revendications 1 et 2, pour la fabrication d'un médicament destiné au traitement de la résistance à l'insuline.

8. Utilisation d'un composé selon une ou plusieurs des revendications 1 et 2, pour la fabrication d'un médicament destiné au traitement de l'obésité pathologique.

9. Procédé pour la fabrication d'un médicament contenant un ou plusieurs des composés selon une ou plusieurs des revendications 1 et 2, **caractérisé en ce qu'**on mélange la substance active avec un véhicule pharmaceutiquement approprié et on met ce mélange sous une forme appropriée à l'administration.
